# EUROPEAN PATENT SPECIFICATION

(11) **EP 1 302 106 B1**
(45) Date of publication and mention of the grant of the patent: **02.02.2005**
(21) Application number: 02022614.8
(22) Date of filing: 09.10.2002
(51) Int. Cl.: A01M 1/20, A61L 9/03

(54) **Heating device for electrodiffuser**
Heizgerät für elektrischen Diffusor
Elément chauffant pour diffuseur électrique

(30) Priority: 15.10.2001 IT MI20012121
(43) Date of publication of application: 16.04.2003
(73) Proprietor: ZOBELE HOLDING S.P.A., 38100 Trento (IT)
(72) Inventor: Zobele, Franco, 38100 Trento (IT); Pedrotti, Andrea, 38070 Pietra Murata (Trento) (IT)
(74) Representative: Petruzziello, Aldo

(56) References cited:
- EP-A- 0 689 766
- EP-A- 0 998 947
- WO-A-02/28443

## Description

The present invention concerns a heating device for an electrodiffuser for solutions, in particular solutions containing active ingredients (principles), such as insecticides, deodorants, disinfectants or similar substances.

The systems currently used for diffusing active ingredients in the air are based on the heating to set temperatures, depending on the type of product to be evaporated, of a porous support containing the product itself. These systems substantially comprise, besides the housing parts and the components for connection to the power system, a container holding the substance to be evaporated and a heating device.

In so far as concerns the parts containing the product, there may be single-dose systems, such as a tablet of porous material soaked with the active ingredient to be diffused, or systems with a refill and a porous element which transfers the liquid from the reserve to the evaporation area. These devices allow several cycles of use and therefore the diffusion of the product over a longer time span.

The heating device, instead, is composed of the source of heat, a resistor with a metal or oxide layer, see e.g. document WO-A-02/28443, wich discloses three resistances connected in series, or a thermistor element (PTC), see e.g. document EP-A-0 998 947, which discloses a PTC element held between two electrodes and an element of ceramic material which ensures electric insulation. This heating part has a high thermal capacity and conduction and therefore allows homogenisation of the temperature with respect to the part containing the product to be evaporated.

The element containing the product is heated either by conduction, when the element itself is in direct contact with the heating body, or by convection: first the air between the heating element and the porous element is heated, then this air heats the porous element.

The heating devices according to the prior art have the principal drawback of not supplying uniform heating of the porous element containing the solution to be evaporated. In fact, due to the thermal characteristics of the materials of which the porous supports currently in use are composed, possible slight variations in shape or position with respect to the heating element may be reflected in different temperatures within the porous elements and thus develop different evaporation conditions of the product as well as possible alterations of the structure of the porous elements themselves.

Another drawback of the heating devices according to the prior art is represented by a certain complexity of structure and assembly of the resistive electric circuit which acts as a heating element. This drawback influences the final cost of the electrodiffuser.

The object of the present invention is to overcome the drawbacks of the prior art, providing a heating device for electrodiffuser which is efficient, effective and able to guarantee uniform heating.

Another object of the present invention is to provide such a heating device for electrodiffusers which is economic and simple to make.

A further object of the present invention is to provide such a heating device for electrodiffuser which is versatile, usable in a modular fashion according to the various additional components, such as a light bulb, switch, timer and the like, and suitable to be applied to various types of electrodiffusers.

These objects are achieved according to the invention with the characteristics listed in the enclosed independent claim 1.

Advantageous embodiments of the invention are given in the dependent claims.

The heating device for electrodiffuser according to the invention comprises a housing shell that contains resistive means which are heated when a current passes through them. In this way the housing shell is heated by heat exchange with the resistive means. The housing shell is in contact with a porous element soaked with active ingredients. In this way, when the porous element is heated by contact with the housing shell, the active ingredients are diffused.

The peculiar characteristic of the invention is represented by the fact that the resistive means comprise at least two electric resistances connected in series by means of a conductive connecting element. This conductive connecting element must have good electric and heat conducting characteristics in order to realise the electric connection of the resistive means and at the same time act also as a heating element for heating the housing shell.

The conductive connecting element is preferably a metal lamina having cuts which engage in the contact terminals of the poles of the resistances. Moreover a pair of metal laminae is provided which engage in the other contact terminals of the poles of the resistances to connect the resistances to the strands of the electric cables of an electric plug for supplying electric power to the resistances.

The advantages of the heating device according to the invention are evident. In fact this device, providing a limited number of elements and connections, is structurally extremely simple and easy to assemble. Moreover the metal laminae serve several purposes and functions, that is:
- connecting the resistances to each other in series and connecting the resistances to the strands of the electric plug;
- blocking the resistances in position inside the housing shell; and
- acting as heating elements to guarantee uniform heating of the housing shell.

Further characteristics of the invention will appear more clearly from the following detailed description which refers to embodiments taken purely as examples and therefore without limitation, illustrated in the enclosed drawings, in which:
Fig. 1 is a perspective view of a first embodiment of a heating device according to the invention;
Fig. 2 is a plan view showing a base half-shell of the heating device in Fig. 1, with fitted inside the electric elements, and an exploded view of a power supply plug;
Fig. 3 is a cross sectional view of the heating device according to the section plane III-III in Fig. 1;
Fig. 4 is a cross sectional view of the base half-shell according to the section plane IV-IV in Fig. 2;
Fig. 5 is a plan view of the internal part of the covering half-shell of the heating device in Fig. 1;
Fig. 6 is a cross sectional view of the covering half-shell, according to the section plane VI-VI in Fig. 5;
Fig. 7 is a cross sectional view , according to the plane VII-VII in Fig. 4, showing the base half-shell and with an exploded view of the electric elements;
Fig. 7A is a cross sectional view showing a pair of laminae according to the section plane A-A in Fig. 7;
Fig. 7B is a cross sectional view showing a lamina according to the section plane B-B in Fig. 7;
Fig. 8 is a part sectional view showing the heating device in Fig. 1 applied to an electrodiffuser of the small bottle type;
Fig. 9 is a top plan view, showing the base half-shell with the assembled electric components of a heating device according to a second embodiment with a luminous element located at the front;
Fig. 10 is a cross sectional view according to the section plane X-X in Fig. 9;
Fig. 11 is a top plan view, showing the base half-shell with the assembled electric components of a heating device according to a variation of the second embodiment with a luminous element located at the rear;
Fig. 12 is a cross sectional view according to the section plane XII-XII in Fig. 11;
Fig. 13 is a top plan view, showing the base half-shell with the assembled electric components of a heating device according to a third embodiment with a luminous element and a timer;
Fig. 14 is a cross sectional view according to the section plane XIV-XIV in Fig. 13;
Fig. 15 is a top plan view, showing the base half-shell with the assembled electric components of a heating device according to a fourth embodiment with a switch;
Fig. 16 is a cross sectional view according to the section plane XVI-XVI in Fig. 15;
Fig. 17 is a perspective view showing a heating device according to a fifth embodiment for an electrodiffuser of the type with a pastille;
Fig. 18 is a top plan view, showing the base half-shell with the assembled electric components of the heating device in Fig. 17;
Fig. 19 is a plan view of the inward facing part of the covering half-shell of the heating device according to the fifth embodiment of the invention;
Fig. 20 is a cross sectional view according to the section plane XX-XX in Fig. 18;
Fig. 21 is a cross sectional view according to the section plane XXI-XXI in Fig. 17
Fig. 22 is a part sectional view illustrating the heating device according to the fifth embodiment applied to an electrodiffuser of the type with a pastille.

With the aid of the Figures the heating device for an electrodiffuser according to the invention is described.

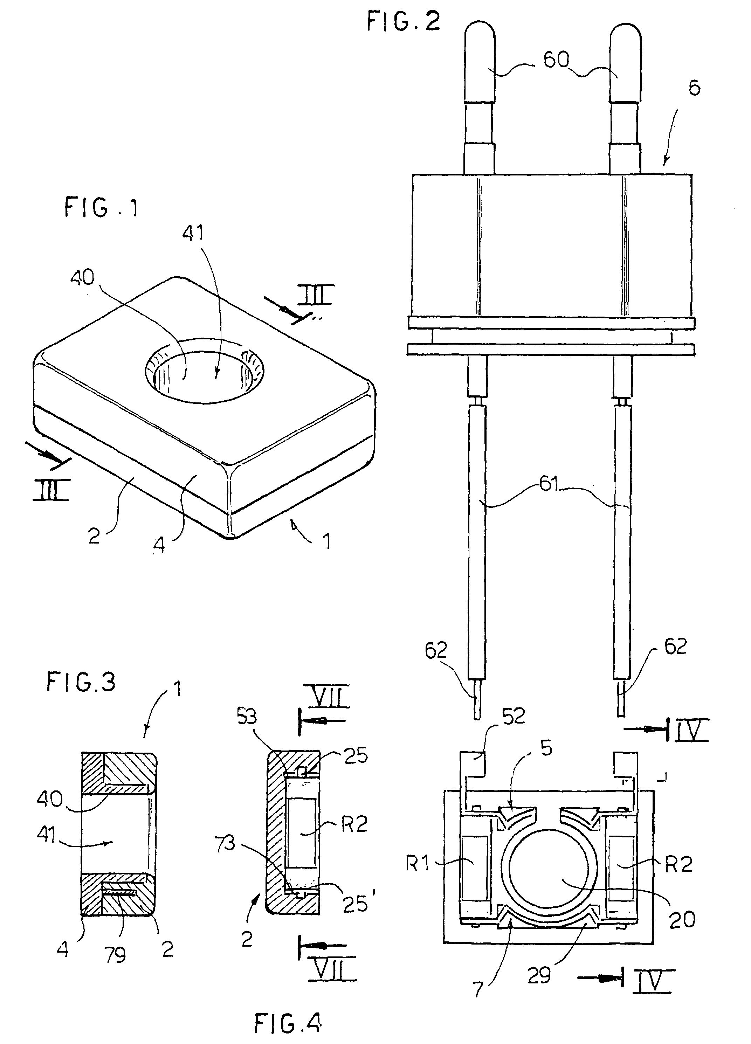

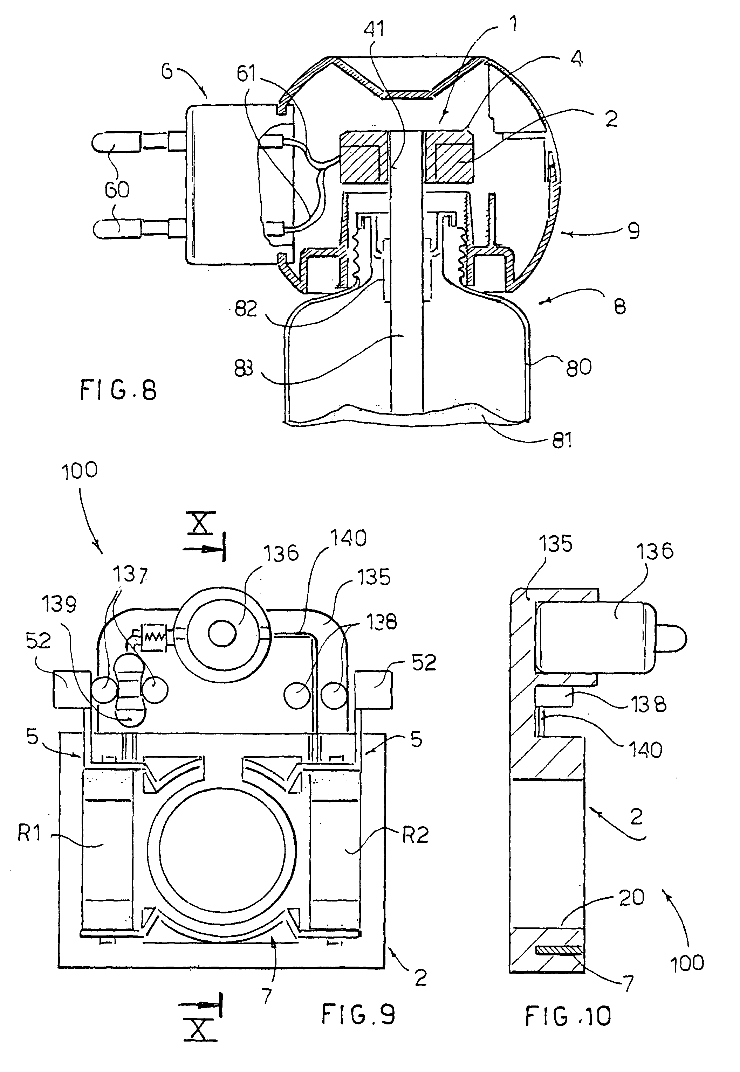

At the moment, with reference to Fig. 1 - 8, a first embodiment of the invention is described, illustrating a heating device, indicated as a whole with the reference numeral 1, suitable to be applied to an electrodiffuser of the small bottle type, shown in Fig. 8 and indicated as a whole with the reference numeral 8.

With reference to Fig. 1, the heating device 1 comprises a box-type body of a substantially parallelepiped shape consisting of a base half-shell 2 and a covering half-shell 4 that can be coupled to the base half-shell 2. Here below the direction along the longer side of the body of the heating device is indicated as the longitudinal direction and the direction along the shorter side of the heating body is indicated as the transverse direction

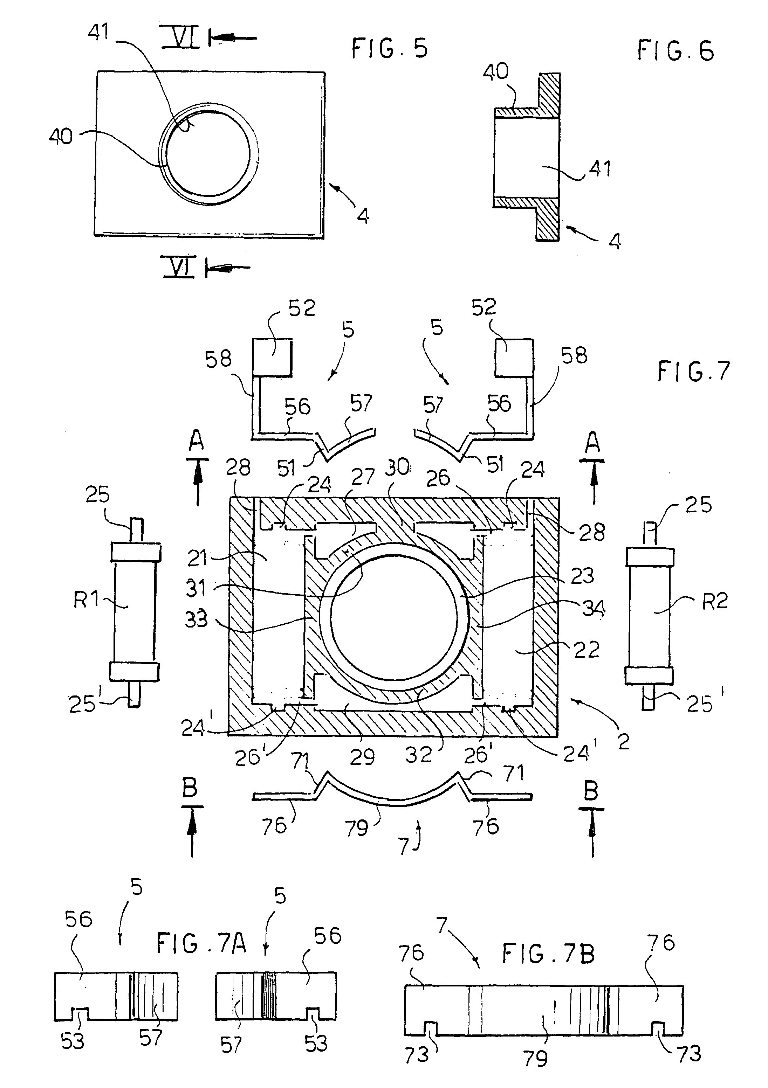

As shown in Fig. 7 the base half-shell 2 has a substantially rectangular plate shape with a circular through hole 20 located in a central position. Around the hole 20 is a thin circular abutting flange 23.

In the body of the base half-shell 2, in positions diametrically opposite to the hole 20, are two transverse housings 21, 22 suited for holding respective electric resistances R1, R2 of a substantially cylindrical shape. The resistances R1 and R2 have a cylindrical central body at the ends of which are two caps from which the respective contact terminals 15, 15' protrude axially. Also in the body of the base half-shell 2, near the opposite ends of each transverse housing 21 and 22 there are two transverse slots 24, 24' communicating with the respective transverse housings 21, 22 and axially positioned with respect to it. Each pair of slots 24, 24' is suited to contain the pair of contact terminals 25, 25' of the poles of each resistance R1 and R2.

The transverse slots 24, 24' cross respective longitudinal slots 26, 26' positioned at right angles to the transverse slots. Each longitudinal slot 26 communicates at one end with a curved seat 27 and at the other end with a transverse slot 28 open towards the outside of the body of the base half-shell 2.

The curved seats 27 are formed in the body of the base half-shell 2, around the circumference of the hole 20, and they define an arc of circumference with a centre coinciding with the centre of the hole 20 and a central angle of slightly below 45°. The two curved seats 27 are separated from each other by a bridge 30 that forms part of the body of the base half-shell 2.

The two inward-facing ends of the two transverse slots 26' communicate with a curved seat 29 that is larger than the curved seats 27 and positioned diametrically opposite to them. The curved seat 29 defines an arc of circumference having a centre coinciding with the centre of the hole 20 and a central angle of slightly below 90°.

It should be noted that the pair of curved seats 27 and the longer curved seat 29 are separated from the central hole 20 by respective thin walls 31 and 32 in the form of an arc of circumference. Also the housings 21 and 22 of the resistances R1 and R2 are separated from the central hole 20 by thin walls 33 and 34.

In each housing 27 is placed a lamina 5 for connection to a power supply plug 6 (Fig. 1). The lamina 5 comprises a curved portion 57 which fits into the curved seat 27. Through a pointed portion 51, the curved portion 57 is connected to a straight longitudinal segment 56 able to be inserted in the longitudinal slot 26. Each longitudinal segment 56 may have a V-shaped groove 53 (Figs. 7A and 4), so as to block in it the respective contact terminal 25 of the pole of the resistance R1, R2, thus ensuring electrical contact and the blocking in position of the resistance R1, R2.

The longitudinal segment 56 ends with a transverse segment 58, lying at a right angle to it. The transverse segment 58 is inserted in the transverse slot 28 and ends with a rectangular plate 52 placed on the outside of the base half-shell 2. As shown in Fig. 2, the strands 62 of the electric cables 61 connected to the poles 60 of the plug 6 are fixed to the respective rectangular plates 52, for example by crimping. Instead of the rectangular plates 52 there may be electrical connectors, for example clips, faston and the like, suitable for holding the strands 62. In any case the electric contact between the strands 62 of the plug 6 and the resistances R1 and R2 is guaranteed by the pair of laminae 5. Alternatively the strands 62 may be inserted directly in the base half-shell 2 and then blocked by the grooves 53 in the pair of laminae 5.

The longer curved seat 29 of the base shell 2 houses a lamina 7 comprising a curved segment 79 suitable to be housed into the curved seat 29. At its ends the curved segment 79 is prolonged by a pointed portion 71, with two straight longitudinal segments 76 suitable to be inserted in the longitudinal slots 26'. Each longitudinal segment 76 may have a respective V-shaped groove 73 (Figs. 7B and 4) which engages in the respective contact terminal 25' of the pole of the resistance R1, R2, thus ensuring electrical contact and the blocking in position of the resistance R1, R2.

It should be noted that the longitudinal segments 56 and 76 of the laminae 5 and 7 are close to, or in close contact with the caps of the resistances R1 and R2, so as to guarantee a better transmission of heat from the resistances R1 and R2 to the laminae 5 and 7 which carry the heat around the hole 20 apt to house the porous element of the electrodiffuser.

This configuration of the base shell and of the components is easily suited for automatic assembly, as the resistances R1 and R2 cut with very short terminals 25, 25' can be manipulated to be inserted in the base half-shell 2 by means of a vibrating feeder. Also the strands 62 of the plug cables can be inserted in the base shell 2 and blocked by the pair of laminae 5, while the resistances R1 and R2 are blocked and connected in series by the connecting lamina 7.

With reference to Figs. 5 and 6, the covering half-shell has the shape of a rectangular plate with the same dimensions as the base half-shell 2. In a central position, the covering half-shell 4 has a cylindrical tang 40 which is hollow on the inside and defines a circular through hole 41. The external diameter of the tang 40 is the same as, or slightly less than the diameter of the hole 20 in the base half-shell, so as to engage in it, as shown in Fig. 3.

The base half-shell 2 and the covering half-shell 4 may be coupled together by welding, for example by ultrasound welding, or by gluing or fixed joint.

The body of the heating device 1 may be made of any electrically insulating material which at the same time offers good heat diffusing characteristics. The base half-shell 2 and the covering half-shell 4 may be made by moulding plastic material, such as PBT (Poly-Butylene-Terephthalate), polyamide, PPS (Poly-Phenyl-Sulphide) and the like.

The laminae 5 and 7 may be made of metal material which has good electricity and heat conducting characteristics, for example copper.

With reference to Fig. 8 the heating device 1 applied to an electrodiffuser of the small bottle type 8 is illustrated. The electrodiffuser 8 comprises a container 80 which contains a liquid solution 81 having active ingredients to be diffused. The container 80 has a neck on which is applied a plug 82 suitable for holding axially, inside the container 80, a cylindrical wick 83 made of porous material. In this way one end of the wick 83 is immersed in the solution 81 inside the container 80 and the other end of the wick 83 protrudes axially from the container 80 to engage in the hole 41 of the covering half-shell 4 of the heating device 1.

The electric contact plates 52 of the laminae 5 are connected to the strands of the electric cables 61 of the electric plug 6. The plug 6 is connected to a support 9, substantially spherical in shape, connected in turn to the neck of the container 80. The support 9 has slots to allow the active ingredients diffused by the porous element to pass outside. In particular, the plug 6 is rotatable fitted on a side wall of the support 9, so that the support 9 can turn around the axis of the plug 6 according to the orientation of the electric socket in which the poles 60 of the plug 6 are to be inserted.

Consequently the passage of electric current through the electric cables 61 of the plug 6 causes current to pass through the pair of laminae 5, through the pair of resistances R1 and R2 connected to the laminae 5, and through the lamina 7 which connects in series the two resistances R1 and R2. As a result the laminae 5 and 7 and the resistances R1 and R2 are overheated, uniformly overheating the wall arches 31, 32, 33, 34 around the hole 20. This results in an exchange of heat between said curved wall parts of the base half-shell and the cylindrical tang 40 of the covering half-shell which is in close contact with the external surface of the wick 83. The final result is that the active ingredients contained in the solution 81 in the container 80 evaporate outside through the surface of the wick 83 which is heated by the heating device 1.

In the description of the following embodiments, the same reference numerals will be used to indicate elements the same as or corresponding to those already described above, and so their detailed description will be omitted.

Figs. 9 and 10 illustrate a second embodiment of the heating device indicated as a whole with the reference numeral 100. The base half-shell 2 of the heating device 100 has a structure substantially the same as that of the base half-shell of the first embodiment. The base half-shell 2 houses two resistances R1 and R2, a pair of laminae 5 bearing the contacts 52 for the strands of the electric plug and a lamina 7 for connecting the two resistances R1 and R2.

The difference compared with the first embodiment lies in the fact that, connected to the base half-shell 2 there is a support plate 135 which protrudes at the front of it (that is it protrudes from the part where the electric contacts 52 protrude, which are intended to engage in the strands of the plug 6).

In the support plate 135 there is a housing suitable for holding a luminous element 136 placed with the source of light facing upwards (at a right angle to the support plate 135). The luminous element 136 may be any type of light bulb, for example a neon light or a LED.

In the support plate 135 there is a first pair of pins 137 for holding one or more electronic components 139, such as a resistance, and a second pair of pins 138 for holding and guiding an electric cable 140. The electric cable 140 connects an electric contact pin of the luminous element 136 to a lamina 5. The ends of the electronic component 139 are connected respectively to the other lamina 5 and to the other electric contact pin of the luminous element 136.

In this way an electronic circuit is obtained, suitable for supplying currents and voltages required for feeding the luminous element 136 from the mains supply taken by the plug 6. So, when the plug 6 is connected to the power mains, the luminous element 136 is fed and gives a luminous signal indicating that the heating device 100 is operating.

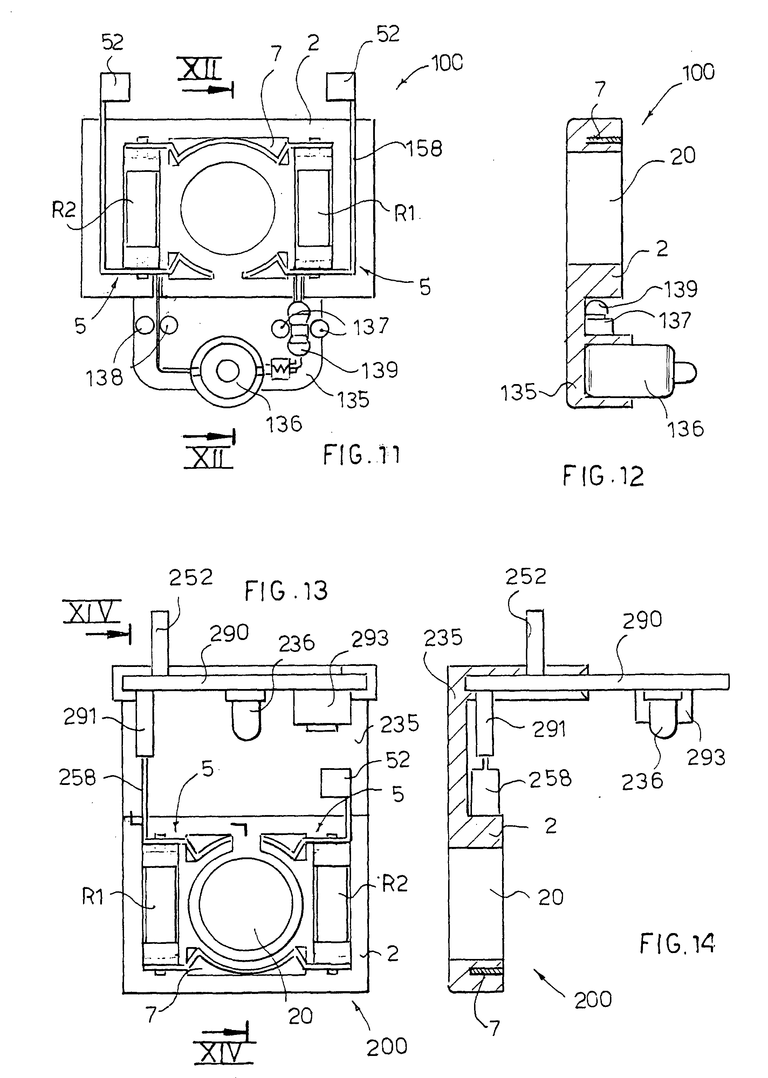

With reference to Figs. 11 and 12, a variation of the second embodiment is described, wherein the base half-shell 2 and the support plate 135 are substantially identical to those of the second embodiment. The only variation lies in the fact that the laminae 5 bearing the electric contacts 52 for the strands of the plug have a long transverse segment 158 which transversely crosses the base half-shell 2 to bring the electric contacts 52 for the strands of the plug from the opposite side with respect to the plate 135 which supports the luminous element 136.

In this way the luminous element 136 is located at the rear with respect to the front part of the heating device 100 wherein there are the electric contacts 52 for the strands of the electric plug.

Figs. 13 and 14 show a heating device 200 according to a third embodiment of the invention, wherein the base half-shell 2 is identical to that of the first embodiment and a support plate 235 having a substantially L-shaped section is connected to it. Fixed to the support plate 235 is a circuit board 290 placed at a right angle to the plain of the base half-shell 2.

A luminous element 236, a timer 293 and the electronic components for the operation of the luminous element and of the timer are mounted on the circuit board 290. One lamina 5 has the contact 52 for the strands of the power supply plug and the other lamina 5 has a transverse segment 258 which is connected to a contact 291 of the circuit board 290. The circuit board 290 has a contact 252 for receiving the strands of a supply cable of the electric plug.

In this way the user, by activating the timer 293, selects the period of operation of the heating device 200 and this operation is indicated by the luminous element 236. Once the selected period of operation has elapsed, the timer 209 interrupts the power supply from the electric plug to the heating device 200, thus interrupting operation of the same.

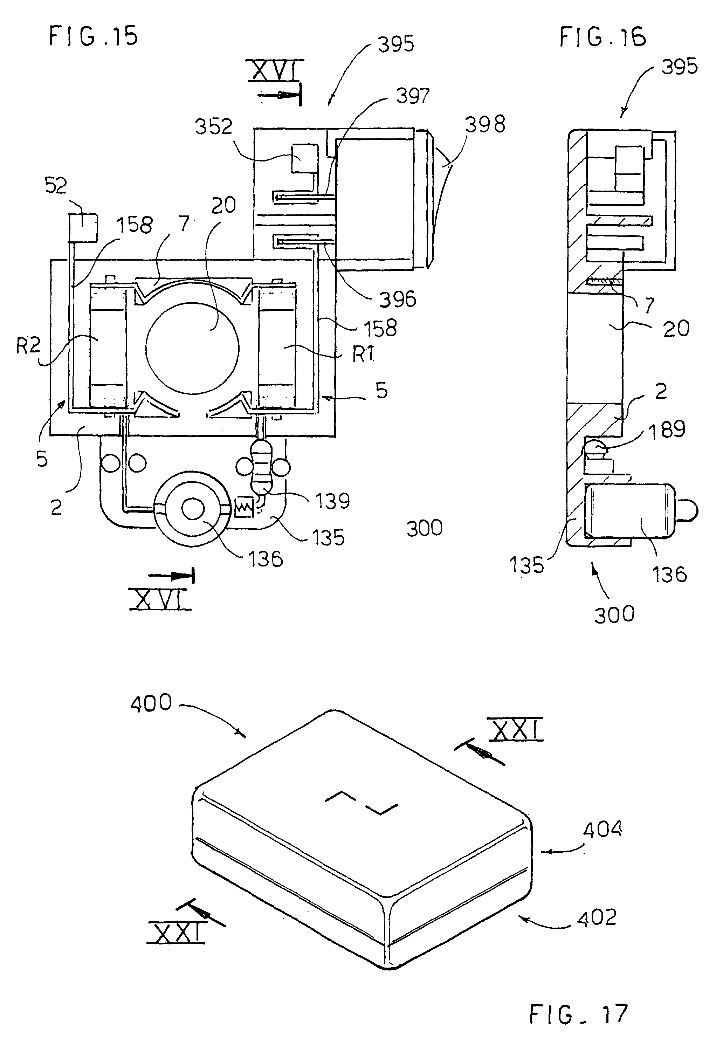

Figs. 15 and 16 show a heating device 300 according to a fourth embodiment of the invention, wherein the base half-shell 2 and the support plate 135 are substantially the same as those illustrated in the variation of the second embodiment, with reference to Figs. 11 and 12. The longitudinal segment 158 of a lamina 5 is connected to a contact 52 located on the part opposite the source of light 136 and intended to be connected to the strand of an electric plug.

The longitudinal segment 158 of the other lamina 5 is connected to one electric contact pin 396 of a switch 395 located on the side of the base half-shell 2 opposite the support plate 135. The other electric contact pin 397 of the switch 395 is connected to an electric contact 352 intended to be connected to the strand of an electric plug.

In this way the switch 395 is interposed between the electric power coming from the electric plug and the resistances R1 and R2 which form the circuit of the heating device. So by pressing the button 398 of the switch the user can enable/disable the operation of the heating device 300.

As regards the second, third and fourth embodiment, to close the respective base half-shells 2 a covering half-shell 4 is used which is substantially identical to the one illustrated in the description of the first embodiment with relation to Figs. 5 and 6.

With the aid of Figs. 17 - 22, a heating device 400, according to a fifth embodiment of the invention, suitable for use in an electrodiffuser 408 of the type with an evaporating pastille 480 is described.

The base half-shell 402 and the covering half-shall 404 of the heating device 400 according to the fifth embodiment differ from the base half-shell 2 and covering half-shell 4 previously described in that the base half-shell 402 does not have the central hole 20 and the covering half-shell 404 does not have the tang 40 for the passage of the porous wick 83.

In this fifth embodiment the pair of laminae 5 for connecting the strands of the plug has a flat plate 457 instead of the curved segment 27 of the previous embodiments and the lamina 7 for connecting the resistances R1 and R2 has a flat plate 479 instead of the curved segment 79. As a result, in the base half-shell 402 there is a pair of rectangular seats 427 for holding the flat plates 457 of the laminae 5 and a rectangular seat 429 for holding the flat plate 479 of the lamina 7.

In this case the covering half-shell 404 has on its inward-facing surface a pair of protruding legs 447 suitable to be abutted against the plates 457 of the pair of laminae 5 for the strands of the electric plug and a protruding leg 449 suitable to be abutted against the plate 479 of the lamina 7 for connecting the resistances.

In this way the heat is transferred uniformly from the plates 457 and 459, through the protruding legs 449 and 447, to the covering half-shell 404. Thus the external surface of the covering half-shell 404, which is intended to hold an evaporating pastille 480, is uniformly heated.

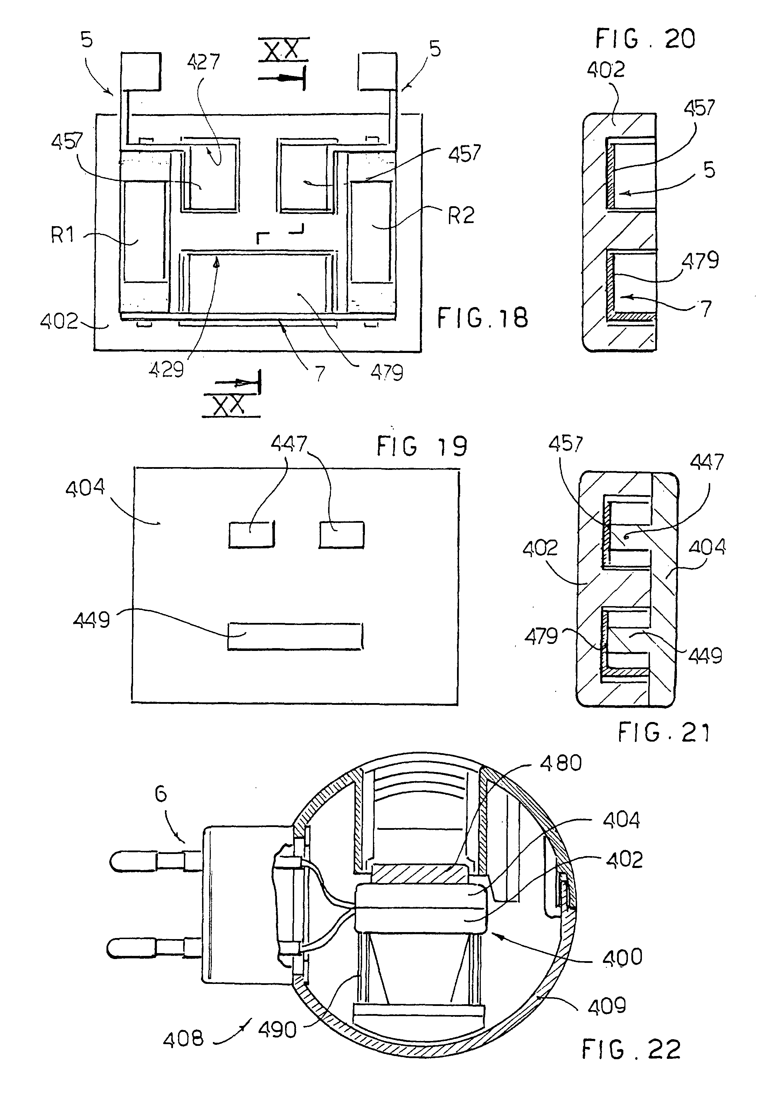

As shown in Fig. 22, the electrodiffuser with pastille 408 comprises an electric plug 6 rotatable mounted on a substantially spherical support 409. Within the support 409 there is another support 490 suitable for supporting the heating device 400 and on the external surface of the covering half-shell 404 a pastille 480 is placed which contains active ingredients that are diffused when it is heated.

Numerous variations may be made to the present embodiments of the invention and detail modifications, within the scope of a skilled in the art, however all falling within the scope of the invention as expressed by the appended claims.

## Claims

1. Heating device (1; 100; 200; 300; 400) for electrodiffuser (9; 409) comprising a housing shell (2, 4; 402, 404) including resistive means (R1, R2) which are heated when a current passes through them in order to heat, by heat exchange, said housing shell which is in contact with a porous element (83; 480) soaked with active ingredients, which are diffused when said porous element is heated,
wherein said resistive means are at least two resistances (R1, R2) connected in series by a conductive connecting element (7) having good characteristics of electricity and heat conduction to realise the electric connection of the resistive means (R1, R2) and, at the same time, to act also as a heating element.

2. Device according to claim 1, **characterised in that** said conductive connecting element (7) is a metal element.

3. Device according to claim 2, **characterised in that** said metal element (7) is in the form of a lamina.

4. Device according to claim 3, **characterised in that** said metal lamina element (7) comprises a pair of grooves (73) suitable to be engaged in the respective contact terminals (25') of said resistive means (R1, R2).

5. Device according to any one of the previous claims, **characterised in that** said housing shell comprises a base half-shell (2; 402) comprising two housings (21, 22) for holding said resistive means (R1, R2) and a seat (29; 429) for holding said connecting conductor element (7), said housing (29; 429) being in communication with the ends of said two housings (21, 22) of the resistive means.

6. Device according to any one of the previous claims, **characterised in that** it comprises a pair of conductive connecting means (5) suitable for connecting the strands (62) of an electric plug (6) with the contact terminals (25) of the poles of said resistances, opposite the poles engaged by said conductive connecting element (7) and suited for acting as heating elements.

7. Device according to claim 6, **characterised in that** said pair of conductive connecting means (5) comprises a pair of metal laminae (5) provided with respective grooves (53) suited for engaging in the contact terminals (25) of the poles of said resistive means (R1, R2).

8. Device according to claim 6 or 7, **characterised in that** said pair of conductive connecting means (5) comprises fixing means (52) suitable for fixing the strands (62) of an electric plug in such a way as to guarantee electric continuity.

9. Device according to any one of the claims from 6 to 8, **characterised in that** the base half-shell (2; 402) further comprises a pair of seats (27; 427) for holding said pair of conductive connecting means (5), said pair of housings (27; 427) not being in communication with each other and being in communication respectively with the ends of said two housings (21, 22) of the resistive means.

10. Device (1; 100; 200; 300) according to any one of the previous claims, **characterised in that** said housing shell (2, 4;) comprises a through hole (41) suitable for holding a porous element (83) of a substantially cylindrical shape for an electrodiffuser of the small bottle type (8).

11. Device according to claim 10, **characterised in that** said housing shell comprises a base half-shell (2) which has a through hole (20) and a covering half-shell (4) comprising a cylindrical tang (40) which can engage in said through hole (20) of the base half-shell, said cylindrical tang (40) of the covering half-shell being hollow on the inside so as to define said through hole (41) for holding said cylindrical porous element (83).

12. Device according to claim 11, **characterised in that** in said base shell (2), said housing (29) for holding the conductive connecting element (7) and said pair of housings (27) for holding the conductive connecting means (5) have a substantially curved shape and are placed around said through hole (20) in said base half-shell.

13. Device according to claim 12, **characterised in that** said connecting conductor element (7) and said conductive connecting means (5) are metal laminae having a substantially curved segment (79, 57) suitable to be housed in said substantially curved seats (29, 27) in said base half-shell and straight segments (76, 56) in which are provided said grooves (73, 53) for engagement in the contact terminals (25', 25) of the resistive means (R1, R2).

14. Device (100) according to any one of the previous claims, or according to claim 5, **characterised in that** it comprises a luminous element (136) electrically connected to said resistive means (R1, R2) or to said pair of conductive connecting means (5) so as to give a luminous signal when an electric current passes through said resistive means.

15. Device according to claim 14, **characterised in that** said luminous element is mounted on a support plate (135) connected to said base half-shell (2), said support plate (135) supporting electric components (139) suitable for supplying a correct electric power supply to the luminous element (136).

16. Device according to claim 14 or 15, **characterised in that** said luminous element (136) is a neon light.

17. Device (200) according to any one of the previous claims, or according to claim 5 **characterised in that** it comprises a timer (293) electrically connected to said resistive means (R1, R2) or to said pair of connecting conductor means (5) so as to allow/interrupt the flow of current from a power supply plug (6) to said resistive means (R1, R2) for a period pre-selected by the user.

18. Device according to claim 17, **characterised in that** said timer (239) is mounted on a circuit board (290) electrically connected to at least one of the connecting conductor means (5) and to at least one strand (62) of the electric plug (6).

19. Device according to claim 18, **characterised in that** on said circuit board (290) is also mounted a luminous element (236) which gives a luminous signal during the period in which a current passes through said resistive means (R1, R2).

20. Device (300) according to any one of the previous claims, or according to claim 5, **characterised in that** it comprises a switch (395) controlled by a button (398) electrically connected to said resistive means (R1, R2) or to said pair of conductive connecting means (5) so as to allow/interrupt the flow of current from a power supply plug (6) to said resistive means (R1, R2).

21. Device (400) according to any one of the claims from 1 to 9, **characterised in that** said porous element (480) containing active ingredients is a pastille (480) placed on an external surface of the shell of said heating device.

22. Electrodiffuser comprising an electric plug (6) fitted on a support body (9; 409) including a heating device (1; 100; 200; 300; 400) in contact with a porous element (83; 480) containing active ingredients which are diffused when the porous element is heated, **characterised in that** it comprises a heating device according to any one of the previous claims.

## Patentansprüche

1. Eine Heizvorrichtung (1; 100; 200; 300; 400) für Elektrodiffusor (9; 409), die eine Gehäuseschale (2; 4; 402; 404) umfasst, in der Widerstandselemente (R1, R2) enthalten sind, die sich aufheizen, wenn Strom durch sie hindurch läuft, um durch Wärmeaustausch die genannte Gehäuseschale zu erhitzen, die sich in Kontakt mit einem porösen Element (83; 480) befindet, das mit Wirkstoffen getränkt ist, die ausströmen, wenn das genannte poröse Element erhitzt wird, wobei die genannten Widerstandselemente mindestens zwei Widerstände sind (R1, R2), die durch ein leitendes Verbindungselement (7) reihengeschaltet sind, das gute elektrische und Wärmeleitfähigkeiten aufweist, um die elektrische Verbindung der Widerstandselemente (R1, R2) herzustellen und gleichzeitig als Heizelement zu wirken.

2. Eine Vorrichtung gemäß Anspruch 1, **dadurch gekennzeichnet, dass** das genannte leitende Verbindungselement (7) ein Metallelement ist.

3. Eine Vorrichtung gemäß Anspruch 2, **dadurch gekennzeichnet, dass** das genannte Metallelement (7) die Form einer Lamelle aufweist.

4. Eine Vorrichtung gemäß Anspruch 3, **dadurch gekennzeichnet, dass** die genannte Metalllamelle (7) ein Paar Rillen (73) aufweist, die geeignet sind, mit den entsprechenden Kontaktklemmen (25') der genannten Widerstandselemente (R1, R2) einzurasten.

5. Eine Vorrichtung gemäß einem beliebigen der vorausgegangenen Ansprüche, **dadurch gekennzeichnet, dass** die genannte Gehäuseschale eine Basishalbschale (2; 402) umfasst, die wiederum zwei Gehäuse (21, 22) umfasst, um die genannten Widerstandselemente (R1, R2) aufzunehmen, und einen Sitz (29; 429), um das genannte leitende Verbindungselement (7) aufzunehmen, wobei das genannte Gehäuse (29; 429) jeweils in Verbindung mit den Enden der genannten zwei Gehäuse (21, 22) der Widerstandselemente steht.

6. Eine Vorrichtung gemäß einem beliebigen der vorausgegangenen Ansprüche,
**dadurch gekennzeichnet, dass** sie ein Paar leitender Verbindungselemente (5) umfasst, die geeignet für die Verbindung der Litzen (62) eines elektrischen Steckers (6) mit den Kontaktklemmen (25) der Pole der genannten Widerstände sind, welche den Polen gegenüberliegen, die mit den genannten leitenden Verbindungselementen (7) einrasten und geeignet sind, als Heizelemente zu wirken.

7. Eine Vorrichtung gemäß Anspruch 6, **dadurch gekennzeichnet, dass** das genannte Paar leitender Verbindungselemente (5) ein Paar Metalllamellen (5) umfasst, die mit entsprechenden Rillen (53) versehen sind, welche geeignet sind, mit den Kontaktklemmen (25) der Pole der genannten Widerstandselemente (R1, R2) einzurasten.

8. Eine Vorrichtung gemäß Anspruch 6 oder 7, **dadurch gekennzeichnet, dass** das genannte Paar leitender Verbindungselemente (5) Feststellelemente (52) umfasst, die geeignet sind, die Litzen (62) eines elektrischen Steckers zu befestigen, und zwar so, dass die elektrische Kontinuität jeweils garantiert wird.

9. Eine Vorrichtung gemäß einem beliebigen der vorausgegangenen Ansprüche von 6 bis 8, **dadurch gekennzeichnet, dass** die Basishalbschale (2; 402) des weiteren ein Paar Sitze (27; 427) zur Aufnahme des genannten Paars leitender Verbindungsmittel (5) umfasst, wobei das genannte Paar Gehäuse (27; 427) nicht miteinander in Verbindung steht und jeweils in Verbindung mit den Enden der genannten zwei Gehäuse (21, 22) der Widerstandselemente steht.

10. Eine Vorrichtung (1; 100; 200; 300;) gemäß einem beliebigen der vorausgegangenen Ansprüche, **dadurch gekennzeichnet, dass** die genannte Gehäuseschale (2; 4) eine durchgehende Öffnung (41) umfasst, die geeignet ist, ein poröses Element (83) von im wesentlichen zylindrischer Form für einen Elektrodiffusor des Fläschchentyps (8) aufzunehmen.

11. Eine Vorrichtung gemäß Anspruch 10, **dadurch gekennzeichnet, dass** die genannte Gehäuseschale eine Basishalbschale (2) umfasst, die eine durchgehende Öffnung (20) und eine Abdeckhalbschale (4) aufweist, welche einen Zapfen (40) umfasst, der in die genannte Öffnung (20) der Basishalbschale einrücken kann, wobei der genannte Zapfen (40) der Abdeckhalbschale innen hohl ist, um die genannte durchgehende Öffnung (41) zur Aufnahme des genannten zylindrischen porösen Elements (83) zu bilden.

12. Eine Vorrichtung gemäß Anspruch 11, **dadurch gekennzeichnet, dass** in der genannten Basisschale (2) das genannte Gehäuse (29) zur Aufnahme des leitenden Verbindungselements (7) und das genannte Paar Gehäuse (27) zur Aufnahme der leitenden Verbindungselemente (5) eine im wesentlichen gekrümmte Form aufweisen und rund um die genannte Öffnung (20) in der genannten Basishalbschale angeordnet sind.

13. Eine Vorrichtung gemäß Anspruch 12, **dadurch gekennzeichnet, dass** das genannte leitende Verbindungselement (7) und die genannten leitenden Verbindungselemente (5) Metalllamellen sind, die ein im wesentlichen gekrümmtes Segment (79, 57) aufweisen, das geeignet ist, in den genannten im wesentlichen gekrümmten Sitzen (29, 27) in der genannten Basishalbschale untergebracht zu werden, und gerade Segmente (76, 56), in denen die genannten Rillen (73, 53) zum Einrasten in die jeweiligen Kontaktklemmen (25', 25) der Widerstandselemente (R1, R2) vorgesehen sind.

14. Eine Vorrichtung (100) gemäß einem beliebigen der vorausgegangenen Ansprüche, bezw. gemäß Anspruch 5, **dadurch gekennzeichnet, dass** sie ein Leuchtelement (136) umfasst, das elektrisch mit den genannten Widerstandselementen (R1, R2) oder mit dem genannten Paar leitender Verbindungselemente (5) verbunden ist, um ein Leuchtsignal zu erzeugen, wenn ein elektrischer Strom durch die genannten Widerstandselemente läuft.

15. Eine Vorrichtung gemäß Anspruch 14, **dadurch gekennzeichnet, dass** das genannte Leuchtelement auf eine Halteplatte (135) montiert ist, die mit der genannten Basishalbschale (2) verbunden ist, wobei die genannte Halteplatte (135) elektrische Bauteile (139) trägt, die geeignet sind, das Leuchtelement (136) mit dem richtigen Netzstrom zu versorgen.

16. Eine Vorrichtung gemäß Anspruch 14 oder 15, **dadurch gekennzeichnet, dass** das genannte Leuchtelement (136) Neonlicht ist.

17. Eine Vorrichtung (200) gemäß einem beliebigen der vorausgegangenen Ansprüche, bezw. gemäß Anspruch 5, **dadurch gekennzeichnet, dass** sie einen Timer (293) umfasst, der elektrisch mit den genannten Widerstandelementen (R1, R2) oder mit dem genannten Paar leitender Verbindungselemente (5) verbunden ist, um den Durchgang des Stroms vom Netzstromstecker (6) zu den genannten Widerstandselementen (R1, R2) während einer vom Benutzer voreingestellten Zeit jeweils zu gestatten/unterbrechen.

18. Eine Vorrichtung gemäß Anspruch 17, **dadurch gekennzeichnet, dass** der genannte Timer (239) auf eine Schaltkarte (290) montiert ist, die elektrisch jeweils mit mindestens einem der leitenden Verbindungselemente (5) und mit mindestens einer Litze (62) des elektrischen Steckers (6) verbunden ist.

19. Eine Vorrichtung gemäß Anspruch 18, **dadurch gekennzeichnet, dass** auf der genannten Schaltkarte (290) ebenfalls ein Leuchtelement (236) montiert ist, das während des Zeitraums, in dem Strom durch die genannten Widerstandselemente (R1, R2) läuft, ein Leuchtsignal erzeugt.

20. Eine Vorrichtung (300) gemäß einem beliebigen der vorausgegangenen Ansprüche, bezw. gemäß Anspruch 5, **dadurch gekennzeichnet, dass** sie einen Schalter (395) umfasst, der durch einen Druckknopf (398) gesteuert wird, welcher elektrisch mit den genannten Widerstandselementen (R1, R2) oder mit dem genannten Paar leitender Verbindungselemente (5) verbunden ist, um den Durchgang des Stroms von dem Netzstromstecker (6) zu den genannten Widerstandselementen (R1, R2) jeweils zu gestatten/unterbrechen.

21. Eine Vorrichtung (400) gemäß einem beliebigen der Ansprüche von 1 bis 9, **dadurch gekennzeichnet, dass** das genannte poröse Element (480), das Wirkstoffe enthält, eine Pastille (480) ist, die an einer äußeren Oberfläche der Schale der genannten Heizvorrichtung angeordnet ist.

22. Ein Elektrodiffusor, der einen elektrischen Stecker (6) umfasst, welcher an einem Haltekörper (9; 409) befestigt ist, der eine Heizvorrichtung (1; 100; 200; 300; 400) in Kontakt mit einem porösen Element (83, 480) umfasst, das Wirkstoffe enthält, die ausströmen, wenn das poröse Element erhitzt wird, **dadurch gekennzeichnet, dass** er eine Heizvorrichtung gemäß einem beliebigen der vorausgegangenen Ansprüche umfasst.

## Revendications

1. Dispositif chauffant (1 ; 100 ; 200 ; 300 ; 400 ) pour diffuseurs électriques (9 ; 409) comprenant une structure d'accueil (2, 4 ; 402, 404) dans laquelle se trouvent des dispositifs de résistance (R1, R2) qui se réchauffent quand ils sont parcourus par un courant de manière à réchauffer, par échange thermique, ladite structure d'accueil qui est en contact avec un élément poreux (83 ; 480) imprégné de principes actifs, qui sont diffusés quand ledit élément poreux est réchauffé, où lesdits dispositifs de résistance sont au minimum deux résistances (R1, R2) reliées en série par un élément conducteur de connexion (7) ayant de bonnes caractéristiques de conductibilité électrique et thermique pour réaliser la connexion électrique des dispositifs de résistance (R1, R2) et, dans un même temps, pour agir également comme un élément chauffant.

2. Dispositif selon la revendication 1, **caractérisé en ce que** ledit élément conducteur de connexion (7) est un élément métallique.

3. Dispositif selon la revendication 2, **caractérisé en ce que** ledit élément métallique (7) a la forme d'une lamelle.

4. Dispositif selon la revendication 3, **caractérisé en ce que** ledit élément métallique à lamelle (7) comprend une paire d'entailles (73) pouvant s'insérer dans les bornes de contact respectives (25') desdits dispositifs de résistance (R1, R2).

5. Dispositif selon une revendication quelconque parmi les revendications susmentionnées, **caractérisé en ce que** ladite structure d'accueil comprend une demi-structure de base (2 ; 402) comprenant deux logements (21, 22) pour accueillir lesdits dispositifs de résistance (R1, R2) et un logement (29 ; 429) pour accueillir ledit élément conducteur de connexion (7), ledit logement (29 ; 429) étant communiquant avec les extrémités desdits deux logements (21, 22) des dispositifs de résistance.

6. Dispositif selon une revendication quelconque parmi les revendications susmentionnées, **caractérisé en ce qu'**il comprend une paire de dispositifs conducteurs de connexion (5) prévus pour relier les torons (62) d'une fiche électrique (6) avec les bornes de contact (25) des pôles desdites résistances, opposés aux pôles engagés par ledit élément conducteur de connexion (7) et prévus pour agir comme des éléments chauffants.

7. Dispositif selon la revendication 6, **caractérisé en ce que** ladite paire de dispositifs conducteurs de connexion (5) comprend une paire de lamelles métalliques (5) pourvues d'entailles respectives (53) prévues pour s'engager dans les bornes de contact (25) des pôles desdits dispositifs de résistance (R1, R2).

8. Dispositif selon la revendication 6 ou 7, **caractérisé en ce que** ladite paire de dispositifs conducteurs de connexion (5) comprend des dispositifs de fixation (52) prévus pour fixer les torons (62) d'une fiche électrique de manière à garantir une continuité électrique.

9. Dispositif selon une revendication quelconque parmi les revendications allant de 6 à 8, **caractérisé en ce que** la demi-structure de base (2 ; 402) comprend également une paire de logements (27 ; 427) pour accueillir ladite paire de dispositifs conducteurs de connexion (5), lesdits deux logements (27 ; 427) n'étant pas en communication entre eux et étant en communication respectivement avec les extrémités desdits deux logements (21, 22) des dispositifs de résistance.

10. Dispositif (1 ; 100; 200; 300) selon une revendication quelconque parmi les revendications susmentionnées, **caractérisé en ce que** ladite structure de logement (2, 4) comprend un orifice de passage (41) prévu pour accueillir un élément poreux (83) à la forme substantiellement cylindrique pour un diffuseur électrique de type à petite boule (8).

11. Dispositif selon la revendication 10, **caractérisé en ce que** la structure d'accueil comprend une demi-structure de base (2) qui possède un orifice de passage (20) et une demi-structure de couverture (4) comprenant une queue cylindrique (40) pouvant s'engager dans ledit orifice de passage (20) de la demi-structure de base, ladite queue cylindrique (40) de la demi-structure de couverture étant creuse à l'intérieur de manière à définir ledit orifice de passage (41) pour accueillir ledit élément cylindrique poreux (83).

12. Dispositif selon la revendication 11, **caractérisé en ce que** ladite structure de base (2), ledit logement (29) pour accueillir l'élément conducteur de connexion (7) et ladite paire de logements (27) pour accueillir les dispositifs conducteurs de connexion (5) ont une forme substantiellement arquée et sont placés autour dudit orifice de passage (20) dans ladite demi-structure de base.

13. Dispositif selon la revendication 12, **caractérisé en ce que** ledit élément conducteur de connexion (7) et lesdits dispositifs conducteurs de connexion (5) sont des lamelles métalliques ayant un segment substantiellement courbe (79, 57) prévues pour être accueillies dans lesdits logements substantiellement courbes (29, 27) dans ladite demi-structure de base et des segments droits (76, 56) où lesdites entailles (73, 53) sont prévues pour l'engagement dans les bornes de contact (25', 25) des dispositifs de résistance (R1, R2).

14. Dispositif (100) selon une revendication quelconque parmi les revendications susmentionnées, ou selon la revendication 5, **caractérisé en ce qu'**il comprend un élément lumineux (136) relié électriquement aux dits dispositifs de résistance (R1, R2) ou à ladite paire de dispositifs conducteurs de connexion (5) de manière à émettre un signal lumineux quand un courant électrique passe à travers lesdits dispositifs de résistance.

15. Dispositif selon la revendication 14, **caractérisé en ce que** ledit élément lumineux est monté sur une plaque de support (135) reliée à ladite demi-structure de base (2), ladite plaque de support (135) soutenant les composants électriques (139) prévus pour fournir une alimentation électrique adéquate à l'élément lumineux (136).

16. Dispositif selon la revendication 14 ou 15, **caractérisé en ce que** ledit élément lumineux (136) est une lampe à néon.

17. Dispositif (200) selon une revendication quelconque parmi les revendications susmentionnées, ou selon la revendication 5, **caractérisé en ce qu'**il comprend une minuterie (293) reliée électriquement aux dits dispositifs de résistance (R1, R2) ou à ladite paire de dispositifs conducteurs de connexion (5) de manière à permettre/interrompre le flux de courant d'une fiche d'alimentation électrique (6) aux dits dispositifs de résistance (R1, R2) pendant une période présélectionnée par l'usager.

18. Dispositif selon la revendication 17, **caractérisé en ce que** ladite minuterie (239) est montée sur une fiche de circuit (290) reliée électriquement à au minimum un desdits dispositifs conducteurs de connexion (5) et à au minimum un toron (62) de la fiche électrique (6).

19. Dispositif selon la revendication 18, **caractérisé en ce qu'**un élément lumineux (236) est également monté sur ladite fiche de circuit (290), lequel fournit un signal lumineux pendant la période où un courant passe à travers lesdits dispositifs de résistance (R1, R2).

20. Dispositif (300) selon une revendication quelconque parmi les revendications susmentionnées, ou selon la revendication 5, **caractérisé en ce qu'**il comprend un interrupteur (395) commandé par un bouton (398) relié électriquement aux dits dispositifs de résistance (R1, R2) ou à ladite paire de dispositifs conducteurs de connexion (5) de manière à permettre/interrompre le flux de courant de la fiche d'alimentation électrique (6) aux dits dispositifs de résistance (R1, R2).

21. Dispositif (400) selon une revendication quelconque parmi les revendications allant de 1 à 9, **caractérisé en ce que** ledit élément poreux (480) contenant des principes actifs est une pastille (480) placée sur une surface extérieure de la structure dudit dispositif chauffant.

22. Diffuseur électrique comprenant une fiche électrique (6) montée sur une structure de support (9 ; 409) comprenant un dispositif chauffant (1 ; 100 ; 200 ; 300 ; 400) en contact avec un élément poreux (83 ; 480) contenant des principes actifs qui sont diffusés quand l'élément poreux est réchauffé, **caractérisé en ce qu'**il comprend un dispositif chauffant selon une revendication quelconque parmi les revendications susmentionnées.
